# EUROPEAN PATENT APPLICATION

(11) **EP 2 679 160 A2**
(43) Date of publication of application: **01.01.2014**
(21) Application number: 12749068.8
(22) Date of filing: 20.01.2012
(51) Int. Cl.: A61B 8/00, G01N 29/24

(54) **ULTRASONIC PROBE HAVING A BONDED CHEMICAL BARRIER**

(30) Priority: 21.02.2011 KR 20110015299
(71) Applicant: Samsung Medison Co., Ltd., Hongcheon-gun, Gangwon-do 250-875 (KR)
(72) Inventor: HA, Yong Soo, Gyeongju-si Gyeongbuk 780-905 (KR); OH, Won Gee, Gyeongju-si Gyeongbuk 780-905 (KR); SONG, In Jin, Gyeongju-si Gyeongbuk 780-905 (KR)
(74) Representative: Schmid, Wolfgang
(86) International application number: PCT/KR2012/000577
(87) International publication number: WO 2012/115355

(57) **Abstract**

Provided is an ultrasound probe including a bonded chemical barrier. The chemical barrier is manufactured between a matching layer and a lens layer by cross-sectionally applying a sputtering layer formed of a nichrome (NiCr) layer and a titanium (Ti) layer onto polyetherimide (PEI) to thereby completely prevent lifting of the lens layer or rising of the matching layer. In addition, as the chemical barrier is attached between the lens layer and the matching layer by using an epoxy adhesive, or a urethane adhesive, the ultrasound probe is fully resistant to long time soaking in an antiseptic, an abstergent, a gel, or the like.

## Description

### Technical Field

The present invention relates to an ultrasound probe with a bonded chemical barrier, and more particularly, to an ultrasound probe that includes a chemical barrier between a matching layer and a lens layer to thereby remarkably reduce the occurrence of defects such as lifting the lens layer or rising of the matching layer.

### Background Art

An ultrasound scanning apparatus is an example of a medical ultrasound apparatus and is mainly used to obtain a contrast image of an organ or a fetus in a human body. Unlike other medical apparatuses for obtaining a contrast image of an inner part of a human body, such as an X-ray imaging apparatus, a computerized tomography (CT) apparatus, and a magnetic resonance imaging apparatus, the ultrasonic scanning apparatus may obtain a contrast image of a desired area inside the human body while a user arbitrarily changes a radiation angle of ultrasound waves. Compared to other medical apparatuses, the ultrasound medical apparatus is advantageous in that the human body is not damaged by ultrasound radiation and also a contrast image of an inner part of the human body is obtained relatively faster.

In order to acquire an image by using an ultrasound scanning apparatus, a unit and/or an apparatus that converts an ultrasound signal into an electric signal and vice versa is necessary. Such a unit is referred to as an ultrasound probe or an ultrasound transducer. The ultrasound probe generally includes a ultrasound module comprising a piezoelectric layer that converts an electrical signal into a sound signal and vice versa as a piezoelectric material therein is vibrated; a matching layer that reduces a difference in an acoustic impedance between the piezoelectric layer and a human body so that a ultrasound wave generated in the piezoelectric layer is transmitted to a target portion of the human body as much as possible; a lens layer that focuses on a predetermined point the ultrasound wave proceeding toward the frontal part of the piezoelectric layer; and a backing layer that blocks the ultrasound wave from proceeding back to the piezoelectric layer to thereby prevent image distortion. Except for a single ultrasound element designed for particular uses, a typical medical ultrasound probe includes a plurality of ultrasound elements.

However, in the medical ultrasound probe, the lens layer attached on the matching layer may be occasionally lifted therefrom during use, which may cause poor transmission or reception of the ultrasound wave. Also, due to a defect of the lens layer or the matching layer, internal components of the medical ultrasound probe may be damaged.

### Disclosure of the Invention

### Technical Problem

The present invention provides an ultrasound probe including a bonded chemical barrier, wherein the chemical barrier by is formed between a lens layer and a matching layer by cross-sectionally applying a sputtering layer of a nichrome (NiCr) layer and a titanium (Ti) layer on polyetherimide (PEI) so that lifting of the lens layer or rising of a matching layer is prevented.

The prevent invention also provides an ultrasound probe including a bonded chemical barrier, wherein the chemical barrier is attached between a lens layer and a matching layer by using an epoxy adhesive or a urethane adhesive so that the ultrasound probe is fully resistant to long-time soaking in an antiseptic, abstergent, gel, or the like.

### Technical Solution

According to an aspect of the present invention, there is provided an ultrasound probe including: a piezoelectric layer that converts an electrical signal into a sound signal and vice versa as a piezoelectric material is vibrated; a matching layer that reduces a difference in an acoustic impedance between the piezoelectric layer and a human body so that an ultrasound wave generated by the piezoelectric layer is transmitted to a target portion of the human body as much as possible; a lens layer that focuses to a predetermined point the ultrasound wave proceeding toward a frontal part of the piezoelectric layer; a backing layer that blocks the ultrasound wave from proceeding back to the piezoelectric layer so as to prevent image distortion; and a chemical barrier disposed between the matching layer and the lens layer to prevent lifting of the lens layer, wherein the chemical barrier includes: a polymer film formed of polyetherimide (PEI); and a sputtering layer that is cross-sectionally deposited on the polymer film and is formed of a nichrome (NiCr) layer and a titanium (Ti) layer.

The polymer film may have a thickness of about 5 µm to about 10 µm.

The nichrome layer may have a thickness of about 50 Å to about 200 Å, and the titanium layer may have a thickness of about 500 Å to about 2000 Å.

The titanium layer may be formed on the nichrome layer.

The polymer film may be attached onto the lens layer.

The chemical barrier may be attached onto the matching layer by using an epoxy adhesive, a urethane adhesive or a silicone adhesive.

### Effects of the Invention

According to the ultrasound probe including the bonded chemical barrier according to the embodiment of the present invention, the chemical barrier is manufactured between a matching layer and a lens layer by cross-sectionally applying a sputtering layer formed of a nichrome (NiCr) layer and a titanium (Ti) layer onto PEI to thereby completely prevent lifting of the lens layer or rising of the matching layer.

In addition, according to the ultrasound probe including the bonded chemical barrier according to the embodiment of the present invention, the chemical barrier is attached between the lens layer and the matching layer by using an epoxy adhesive or a urethane adhesive. Thus, the ultrasound probe is fully resistant to long-term soaking in an antiseptic, abstergent, gel, or the like.

### Brief Description of the Drawings

The above and other features and advantages of the present invention will become more apparent by describing in detail exemplary embodiments thereof with reference to the attached drawings in which:
FIG. 1 is a disassembled perspective view of an ultrasound module of a typical ultrasound probe; and
FIG. 2 is a cross-sectional view of an ultrasound probe including a bonded chemical barrier, according to an embodiment of the present invention.

### < Explanation of Reference Numerals>

10: ultrasound probe bonded with a chemical barrier
100: chemical barrier 110: polymer film
120: sputtering layer 121: nichrome layer
122: titanium layer

### Best Mode for carrying out the Invention

Hereinafter, the present invention will now be described more fully with reference to the accompanying drawings, in which exemplary embodiments of the present invention are shown such that one of ordinary skill in the art may easily work the invention. In the description of the present invention, certain detailed explanations of related art are omitted when it is deemed that they may unnecessarily obscure the essence of the invention. Like reference numerals denote like elements throughout.

In addition, in the present specification, when a constituent element "contacts" or is "connected" to other constituent element, the constituent element contacts or is connected to the other constituent element not only directly but also "indirectly" through at least one other constituent element interposed therebetween. Also, when a part may "include" a certain constituent element, unless specified otherwise, it may not be construed to exclude another constituent element but may be construed to further include other constituent elements.

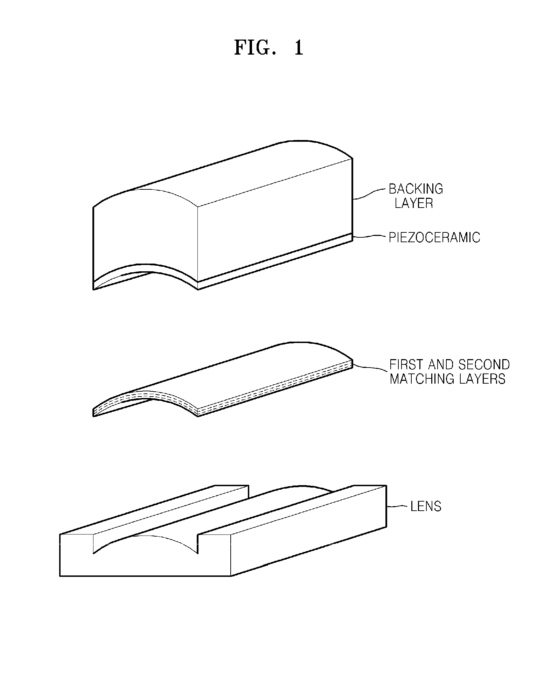

FIG. 1 is a disassembled perspective view of an ultrasound module of a typical ultrasound probe. As illustrated in FIG. 1, a typical ultrasound probe includes an ultrasound module that transmits or receives an ultrasound wave, and the ultrasound module includes a backing layer, a piezoelectric layer (piezoceramic layer), a matching layer, and a lens layer.

In the typical ultrasound probe, the lens layer may be separated from the matching layer or the lens layer may occasionally expand. In this case, an ultrasound wave may not be easily transmitted or received, and thus, an exact image of an inner area of a human body may not be provided.

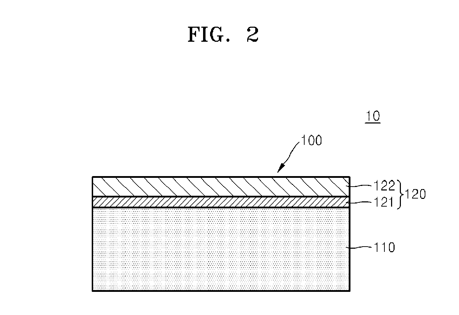

FIG. 2 is a cross-sectional view of an ultrasound probe including a bonded chemical barrier, according to an embodiment of the present invention. As illustrated in FIG. 2, the ultrasound probe with the bonded chemical barrier according to the current embodiment of the present invention may include a piezoelectric layer (not shown) that converts an electric signal into a sound signal and vice versa as a piezoelectric material that is vibrated, a matching layer (not shown) that reduces a difference in an acoustic impedance between the piezoelectric layer and a human body so that a ultrasound wave generated by the piezoelectric layer is transmitted to a target portion of the human body as much as possible, a lens layer (not shown) that focuses to a predetermined point the ultrasound wave proceeding to the frontal part of the piezoelectric layer; a backing layer (not shown) that blocks the ultrasound wave from proceeding back towards the piezoelectric layer to thereby prevent image distortion, and a chemical barrier 100. The piezoelectric layer, the matching layer, the lens layer, and the backing layer are the same as those used in typical ultrasound probes, and thus, detailed descriptions thereof will be omitted.

The chemical barrier 100 may be interposed between the matching layer and the lens layer to prevent lifting of the lens layer. According to an embodiment of the present invention, the chemical barrier 100 is applied between the matching layer and the lens layer to thereby completely prevent lifting of the lens layer from the matching layer. The chemical barrier 100 may include a polymer film 110 and a sputtering layer 120. The polymer film 110 may be formed of polyetherimide (PEI). The polymer film 110 may have a thickness of about 5 µm to about 10 µm (in detail, 7 µm), and may be attached onto the lens layer.

The sputtering layer 120 may be cross-sectionally deposited on the polymer film 110, and may be formed of a nichrome (NiCr) layer 121 and a titanium (Ti) layer 122. The nichrome layer 121 may have a thickness of about 50 Å to 200 Å (in detail, 100 Å), and the titanium layer 122 may have a thickness of about 500 Å to about 2000 Å (in detail, 1000 Å), and the titanium layer 122 may be formed on the nichrome layer 121.

In the ultrasound probe including the chemical barrier 100, according to the current embodiment of the present invention, the chemical barrier 100 is attached to each of the matching layer and the lens layer. The chemical barrier 100 may be attached to the matching layer by using an epoxy, urethane, or silicone adhesive. However, the present invention is not limited to the above-described attaching method.

For example, a lens material may be attached on the chemical barrier 100 so that the lens layer may be immediately formed on a surface of the chemical barrier. Accordingly, according to the ultrasound probe including the bonded chemical barrier according to the current the embodiment of the present invention, lifting of the lens layer may be prevented.

The ultrasound probe including the bonded chemical barrier according to the current embodiment of the present invention as described above, the chemical barrier 100 formed of PEI, nichrome, and titanium is disposed between the matching layer and the lens layer, and the lens layer is attached onto the chemical barrier to thereby prevent lifting of the lens layer. In addition, if an antiseptic, abstergent, gel, or the like contacts the ultrasound probe, penetration thereof between the lens layer and the matching layer is prevented, and thus, the ultrasound probe has excellent resistance to long-term soaking in such materials.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. An ultrasound probe comprising:
a piezoelectric layer that converts an electrical signal into a sound signal and vice versa as a piezoelectric material is vibrated;
a matching layer that reduces a difference in an acoustic impedance between the piezoelectric layer and a human body so that an ultrasound wave generated by the piezoelectric layer is transmitted to a target portion of the human body as much as possible;
a lens layer that focuses to a predetermined point the ultrasound wave proceeding toward a frontal part of the piezoelectric layer;
a backing layer that blocks the ultrasound wave from proceeding back to the piezoelectric layer so as to prevent image distortion; and
a chemical barrier disposed between the matching layer and the lens layer to prevent lifting of the lens layer,
wherein the chemical barrier comprises:
a polymer film formed of polyetherimide (PEI); and
a sputtering layer that is cross-sectionally deposited on the polymer film and is formed of a nichrome (NiCr) layer and a titanium (Ti) layer,
wherein the chemical barrier is attached onto the matching layer by using an epoxy, urethane, or silicone adhesive.

2. The ultrasound probe of claim 1, wherein the polymer film has a thickness of about 5 µm to about 10 µm.

3. The ultrasound probe of claim 1, wherein the nichrome layer has a thickness of about 50 Å to about 200 Å, and the titanium layer has a thickness of about 500 Å to about 2000 Å.

4. The ultrasound probe of claim 1, wherein the titanium layer is formed on the nichrome layer.

5. The ultrasound probe of claim 1, wherein the polymer film is attached onto the lens layer.
